# EUROPEAN PATENT SPECIFICATION

(11) **EP 0 283 075 B1**
(45) Date of publication and mention of the grant of the patent: **12.05.1999**
(21) Application number: 88200377.5
(22) Date of filing: 29.02.1988
(51) Int. Cl.: C12N 15/75, C12N 15/57, C12N 1/20, C12N 9/54, C12Q 1/68

(54) **Molecular cloning and expression of genes encoding proteolytic enzymes**
Molekulare Klonierung und Expression von proteolytische Enzyme kodierenden Genen
Clonage moléculaire et expression de gènes codant pour des enzymes protéolytiques

(30) Priority: 27.02.1987 EP 87200358
(43) Date of publication of application: 21.09.1988
(60) Divisional application: 91203297.6
(73) Proprietor: GENENCOR INTERNATIONAL, INC., Rochester, New York 14618 (US)
(72) Inventor: Van Eekelen, Christian Albertus Gerardus, NL-2661 HD Bergschenhoek (NL); Van der Laan, Johannes Cornelis, NL-1062 CP Amsterdam (NL); Mulleners, Leonardus Johannes Sofie Marie, NL-5121 SV Rijen (NL)
(74) Representative: Armitage, Ian Michael

(56) References cited:
- EP-A- 0 108 301
- EP-A- 0 130 756
- EP-A- 0 134 048
- EP-A- 0 138 075
- EP-A- 0 251 446
- WO-A-86/01825
- WO-A-87/04461
- FR-A- 2 292 042
- APPL BIOCHEM MICROBIOL , vol.14, no.10, 1978/9, page 511 - 523, ''
- APPL MICROBIOL BIOTECHNOL, vol.34, no., 1990, page 57 - 62, ''
- AGRIC BIOL CHEM, vol.48, no.2, 84, page 397 - 401, ''
- CRIT REV BIOTECHNOL, vol.3, no.3, 1986, page 199 - 234, ''
- Gene, Vol. 30, No. 1052, 1984, pages 17-22; Elsevier Sc. Publ.; V.A. CORFIELD et al.: "A modified protoplast-regeneration protocol facilitating the detection of cloned exoenzyme genes in Bacillus subtilis"
- Biological Abstracts, Vol. 82, No. 9, 1986, page 871, Ref. No. 86427; Philadelphia, US, Y.J. HYUN et al.: "Physiological properties and transformaties of alkaline-tolerant bacteria", & Korean J. Appl. Microbiol. Bioeng, 14(3), 239-244, 1986
- Agricultural and Biological Chemistry, Vol. 47, No. 9, September 1983, pages 2101-2102; Tokyo, JP, R. USAMI et al.: "Plasmids in alkalophilic Bacillus sp."
- Proc. Natl. Acad. Sci. USA, Vol. 81. No. 4, February 1984, pages 1184-1188; Washington, US, S.-L. WONG et al.: "The subtilisin E gene of Bacillus subtilis is transcribed from a sigma-37 promoter in vivo", page 1187.
- Nature, Vol. 318, November 28, 1985, pages 375-376, P.G. THOMAS et al.: "Tailoring the pH dependance of enzyme catalysis using protein engineering", Figure 1.
- Biological Abstracts/RRM, Ref. No. 34060253; Philadelphia, US, C. PAK et al.: "Cloning of protease gene produced by heat-resistant alkalinic Bacillus in Escherichia coli", & Abstract Biotechnology Genetic Engineering, whole Abstract.
- Biological Abstracts/RRM, Ref. No. 85 003592; Philadelphia, US, B.C. CUNNINGHAM et al.: "Improvement in the alkaline stability of subtilisin using an efficient random mutagenesis and screening procedure", & Protein Eng 1987, 1, (4), 319-326, whole Abstract.
- Spec. Publ. Soc. Gen. Microbiol., Vol. 17, "Microbesd Extreme Environ", 1986, pages 297-315: chapter 10, K. HORIKOSHI: "Genetic applications of alkalophilic microorganisms", whole article.

## Description

### Technical Field

The field of this invention relates to enhanced production of serine protease in Bacillus strains employing recombinant techniques.

### Background

Bacilli have been used widely for the production of industrially important enzymes such as alpha-amylase, neutral protease and alkaline or serine proteases. In general, bacterial serine proteases can be obtained from many prokaryotic organisms including gram negative organisms such as Serratia or Pseudomonas species and gram positive bacteria such as Micrococcus and Bacillus species. A group of industrially important serine proteases of particular interest are those which have high activity in alkaline media. While industrial serine proteases are produced by various Bacillus species, including B. subtilis, B. licheniformis, B. amyloliquefaciens, B. alcalophilus and other Bacillus species, the high alkaline proteases are generally produced by Bacillus strains which are capable of growing at alkaline pH. An example of such a bacillus is Bacillus novo species PB92.

There is substantial interest in developing strains of bacilli capable of producing proteolytic enzymes in high yield, particularly high alkaline proteases.

### Relevant Literature

Several genes for extracellular enzymes of bacilli have been successfully cloned, such as the alpha-amylase genes of B. amyloliquefaciens (Palva et al., Gene 15 (1981) 43-51), B. licheniformis (Ortlepp, Gene 23 (1983) 267), B. stearothermophilus (Mielenz et al., Proc. Natl. Acad. Sci. USA 80 (1983) 5975-5979; EPA-0057976) and B. subtilis (Yang et al., Nucleic Acids Res. 11 (1983) 237); the levansucrase gene of B. subtilis (Gay et al., J. Bacteriol. 153 (1983) 1424); the neutral protease encoding genes of B. stearothermophilus (Fuji et al., J. Bacteriol. 156 (1983) 831), B. amyloliquefaciens (Honjo et al., J. Biotech. 1 (1984) 165) and of B. subtilis (Yang et al., J. Bacteriol. 160 (1984) 115; the serine or alcaline protease encoding genes of B. subtilis (Wong et al., Proc. Natl. Acad. Sci. USA 81 (1984) 1184), B. licheniformis (Jacobs et al., Nucleic Acids Res. 13 (1985) 8913) and B. amyloliquefaciens (Wells et al., Nucleic Acids Res. 11 (1983) 7911).

Production of industrial serine proteases in various Bacillus species are described in, for example, U.S. Patent Nos. 3,674,643; 3,723,250; and 4,480,043. Production of high alkaline proteolytic enzyme by Bacillus strains capable of growing at alkaline pH are described in, for example, U.S. Patent Nos. 3,723,250; Re. 30,602 and 4,480,037. For a review article of the use of bacilli for production of industrially important enzymes see, for example, Debabov, "The Industrial Use of Bacilli", in: The Molecular Biology of Bacilli, (Acad. Press, New York, 1982).

A protocol for protoplast transformation of B. subtilis was described by Chang and Cohen (Mol. Gen. Genet. 168 (1979) 111-115). Similar protocols have been described for the successful transformation of B. megaterium protoplasts (Vorobjeva et al., FEMS Microbiol. Letters 7 (1980) 261-263), B. amyloliquefaciens protoplasts (Smith et al., Appl. and Env. Microbiol. 51 (1986) 634), B. thuringiensis protoplasts (Fisher et al., Arch. Microbiol. 139 (1981) 213-217), B. sphaericus protoplasts (McDonald, J. Gen. Microbiol. 130 (1984) 203), and B. larvae protoplasts (Bakhiet et al., Appl. and Env. Microbiol. 49 (1985) 577). However, Bakhiet et al. (supra.) reported unsuccessful results with B. popillae. Mann et al., Current Microbiol. 13 (1986) 131-135 reported successful transformation with B. polymyxa, B. licheniformis, B. macerans and B. laterosporus. However, the protocol was not successful with B. coagulans, B. cereus and B. pumilus even though good protoplast formation was observed. Other methods for introducing DNA into protoplasts include fusion with DNA containing liposomes, Holubova, Folia Microbiol. 30 (1985) 97.

The present invention provides an expression cassette comprising:
as operably joined components, (a) a transcriptional regulatory region and a translational initiation region functional in a Bacillus host cell; (b) a DNA sequence encoding a high alkaline serine protease, whose sequence has at least 70% homology to the following amino acid sequence: 
H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH;
 (c) a translational and transcriptional termination region functional in said host cell, wherein expression of said DNA sequence is regulated by said initiation and termination regions; and (d) at least one of a marker gene and a temperature-sensitive origin of replication of a bacterial plasmid, whereby the high alkaline protease has an optimum activity at pH value higher than 9 and retain at least 80% of the optimal activity at a pH of 11 or higher and is obtainable from an alkalopilic Bacillus strain.

The invention further provides a transformed Bacillus host cell capable of producing high-alkaline serine protease comprising said expression cassette, and a method for transforming Bacillus host cells which comprises providing said expression cassette; combining a protoplast prepared from a Bacillus host cell with said cassette in the presence of a fusogen at high pH, whereby said cassette is introduced into said host cell and nucleic acid encoding said protease is integrated into the chromosome of said host cell; and
selecting host cells stably containing said gene and which are capable of producing high alkaline protease, whereby the high alkaline protease has an optimum activity at pH value higher than 9 and retain at least 80% of the optimal activity at a pH of 11 or higher and is obtainable from an alkalopilic Bacillus strain.

The invention further provides the use of a transformed Bacillus host of the invention for the production of a high alkaline serine protease, and a method for producing a high alkaline serine protease in Bacillus, said method comprising:
growing a Bacillus culture comprising Bacillus cells of the invention, and optionally isolating said expression product.

### BRIEF DESCRIPTION OF THE DRAWINGS

Figure 1 shows the results of histidine/MOPS gel electrophoresis performed on supernatants from cultures of B. subtilis DB104 containing pUBllO and pM58, respectively, compared with several subtilisins.
lane 1: Carlsberg subtilisin
lane 2: Bacillus PB92 protease
lane 3: Bacillus subtilis subtilisin
lane 4: Bacillus subtilis DB104 (pM58)
lane 5: Bacillus subtilis DB104 (pUB110)

Figure 2 shows tile restriction map of plasmid pM58. Furthermore, the sequencing strategy is shown in the upper part of the Figure. The arrowed solid lines represent the fragments cloned in the phage M13 vectors mp10, mpll and mp 18. The lower part of the Figure shows the sequencing strategy using 10 oligonucleotides located at regular distances on the protease gene.

Figure 3 shows the nucleotide sequence of the coding strand correlated with the amino acid sequence of the Bacillus PB92 serine protease. Promoters (P₁, P₂), ribosome binding site (rbs) and termination regions (term) of the DNA sequence are also shown. The numbered solid lines represent the location of the ten oligonucleotides used for sequencing.

Figure 4A shows the construction of plasmid pE194-neo.

Figure 4B shows the construction of plasmid pMAX-4.

### DESCRIPTION OF THE SPECIFIC EMBODIMENTS

In accordance with the subject invention, novel DNA constructs and Bacillus strains, for producing proteolytic enzymes in high yield, as well as methods for their preparation are provided. Host cells are transformed by combining protoplasts prepared from the host Bacillus strain under fusing conditions with a plasmid construct comprising a DNA sequence encoding a proteolytic enzyme.

High alkaline proteases are serine proteases or subtilisins with high activity in alkaline media. More specifically, high alkaline proteases have an optimal activity at pH values higher than about 9 and retain at least 80% of this optimal activity at a pH of about 11 or even higher. High alkaline proteases are generally produced by Bacillus strains which are capable of growing at alkaline pH.

The techniques used in isolating the protease gene are known in the art, including synthesis, isolation from genomic DNA, preparation from cDNA, or combinations thereof. Isolation and expression of high alkaline protease genes are disclosed in the priority document upon which this application is based, European Application No. EP-A-87200358.7, which disclosure is incorporated herein by reference. The various techniques for manupulation of the genes are well known, and include restriction, digestion, resection, ligation, in vitro mutagenesis, primer repair, employing linkers and adapters, and the like. See Maniatis et al., Molecular Cloning, Cold Spring Harbor Laboratory, Cold Spring Harbor, New York, 1982.

Generally, the method comprises preparing a genomic library from an organism expressing a high alkaline protease. The genome of the donor microorganism is isolated and cleaved by an appropriate restriction enzyme. The resulting DNA fragments of the donor strain are then ligated into a cloning vector which has been cleaved with a compatible restriction endonuclease.

Clones containing the inserted DNA fragment may be identified by means of a resistance maker or by using a direct or positive selection procedure such as that developed for B. subtilis (Gryczan and Dubnow, Gene 20 (1982) 459-469). In addition, clones expressing a high alkaline protease may be identified by the use of antibodies directed to the expression product, or detection of loss of substrate or formation of product of the proteolytic enzyme. For example, colonies expressing a marker gene such as antibiotic resistance may be screened for protease production as determined by increased precipitation of a halo of caseine around colonies plated on agar plates containing caseine.

Once a complete gene has been identified, either as cDNA or chromosomal DNA, it may then be manipulated in a variety of ways to provide for expression. Bacillus hosts may be employed which include, for example, alkalophilic bacteria, including those alkalophilic bacilli which are already high alkaline protease producers. A preferred host organism is Bacillus novo sp. PB92. It is therefore convenient to maintain the wild-type sequence with regulatory signals and secretory leader sequences, etc., although control regions derived from other bacilli which are functional in the Bacillus host strain can also be used. A suitable vector for transforming a Bacillus cell thus includes a structural gene encoding a high alkaline protease obtainable from an alkalophilic Bacillus including control regions such as a promoter sequence, a sequence forming the ribosomal binding site and sequences controlling termination of transcription and translation of the alkaline protease gene, said control regions being functional in an alkalophilic Bacillus host cell.

The vector additionally may include a marker gene, for example for conferring resistance to an antibiotic to which the host strain is sensitive, and an origin of replication that is capable of replicating autonomously in the host cell. The origin of replication may be one having a mutation which makes its functioning temperature-sensitive in the host cell, thus enabling selection for chromosomal integration as described by Ehrlich, Proc. Natl. Acad. Sci. USA 75 (1978) 1433.

The amino acid sequence can be used to design a probe to screen a cDNA or a genomic library prepared from mRNA or DNA from cells of interest as donor cells for a high alkaline protease gene. By using the high alkaline protease cDNA or a fragment thereof as a hybridization probe, structurally related genes found in other organisms can easily be cloned. Of particular interest is the isolation of genes from organisms that express high alkaline serine proteases by using oligonucleotide probes based on the nucleotide sequences of high alkaline serine protease genes obtainable from alkalophilic Bacillus strains, including Bacillus novo species PB92. Such probes can be considerably shorter than the entire sequence but should be at least 10, preferably at least 15, more preferably 20 nucleotides in length. Longer oligonucleotides are also useful, up to the full length of the gene, preferably no more than 1200 nucleotides in length. Both RNA and DNA probes can be used.

In use, the probes are typically labeled in a detectable manner (e.g., with ³²P, ³H, biotin, or avidin) and are incubated with single-stranded DNA or RNA from the organism in which a gene is sought. Hybridization is detected by means of the label after single-stranded and double-stranded (hybridized) DNA (or DNA/RNA) have been separated (typically using nitrocellulose paper). Hybridization techniques suitable for use with olignucleotides are well known to those skilled in the art.

Although probes are normally used with a detectable label which permits easy identification, unlabeled oligonucleotides are also useful, both as precursors of labeled probes and for use in methods that provide for direct detection of double-stranded DNA (or DNA/RNA). Accordingly, the term oligonucleotide probe refers to both labeled and unlabeled forms. The ligation mixture is then used to transform competent B. subtilis cells. Using the marker gene as a means of selection, recombinant plasmids can then be isolated and characterized by restriction analysis and sequencing.

For high alkaline protease production, a preferred DNA sequence for use in transforming Bacillus strains which already produce serine protease is a sequence originating from Bacillus novo species PB92. The amino acid sequence of the serine protease encoded by the DNA sequence is as follows:

H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH.

While the preferred strain for providing the protease gene is Bacillus novo species PB92, essentially the same serine protease may be obtained from other alkalophilic bacilli. Such protease genes, having at least about 70% and preferably from about 80% homology with the amino acid sequence of Bacillus PB92, are to be understood as within the scope of the present invention.

It is desirable that the expression product be secreted. Since the alkaline protease is secreted, the wild-type secretory leader signals and processing signals can be used. In addition, a fused gene may be prepared by providing a 5' sequence to the structural gene which encodes a secretory leader and a processing signal. Illustrative heterologous secretory leaders include the secretory leaders of Bacillus amylase and protease genes. By fusion in proper reading frame of the secretory leader with the structural gene of interest, the mature alkalophilic protease may be secreted into the culture medium.

The expression cassette may be wholly or partially derived from natural sources, and either wholly or partially derived from sources homologous to the host cell, or heterologous to the host cell. The various DNA constructs (DNA sequences, vectors, plasmids, expression cassettes) of the invention are isolated and/or purified, or synthesized and thus are not "naturally-occurring".

Depending upon whether integration of the structural gene into the chromosome of the host strain or maintenance on an extrachromosomal element is desired, a replication system for Bacillus may or may not be included. For integration, stretches of homology in the plasmid construct with the Bacillus genome may be used to enhance the probability of recombination. Furthermore, inclusion of a temperature-sensitive origin of replication in the plasmid construct permits selection for chromosomal integration. See for example, Ehrlich, Proc. Natl. Acad. Sci. USA 75 (1978) 1433.

More than one copy of the structural gene can be inserted into the host cell to amplify expression of the structural gene. Stable amplification of the structural gene can be obtained by integrating at least one additional copy of the structural gene into the host cell genome and selecting for transformants in which copies of the structural gene are separated by endogenous chromosomal sequences. DNA constructs and methods for prokaryotic systems are described in European Application No. EP-A-87200356.1, which disclosure is incorporated herein by reference.

In addition to the replication system, there will usually be at least one marker gene included in the plasmid construct. By marker gene is intended a structural gene capable of expression in a host which provides for biocide or viral resistance, or resistance to heavy metals, immunity, and the like. For biocide resistance, this may include resistance to antibiotics, for example, neomycin, kanamycin, tetracycline, etc. The marker gene will be selected to provide selection at low copy number at an antibiotic concentration which does not seriously impede growth of recombinant cells. Of particular interest is resistance to nemycin.

The various DNA sequences may be derived from diverse sources and joined together to provide for a vector which includes one or more convenient, preferably unique, restriction sites to allow for insertion or substitution of the structural genes at such sites to provide the plasmid construct.

Once the plasmid construct has been prepared, it can be used for transforming an appropriate host cell. The host Bacillus may be any Bacillus strain, however choice of an appropriate strain takes into account factors which can improve production of high alkaline proteases. Production can be improved in a variety of ways, including using a host in which there is reduced degradation of the desired product, recognition of regulatory signals, ease of secretion, etc. Thus, while a preferred host Bacillus is a high alkaline protease producer, either a wild-type Bacillus or a mutant Bacillus, the host Bacillus can also be a high alkaline protease producer mutant which does not produce high alkaline protease.

High alkaline protease producing bacilli are taxonomically not well classified and are generally referred to as alkalophilic Bacillus strains. Examples of Bacillus strains capable of growing at alkaline pH are described in, for example, US Patent Nos. 3,723,250; Re. 30,602 and 4,480,037. An example of a preferred alkalophilic Bacillus host strain is strain Bacillus novo species PB92 disclosed inter alia in US Patent No. Re. 30,602.

Industrial alkalophilic Bacillus strains can also be used as host cells. Industrial Bacillus strains originate from organisms which may be isolated in the soil or are available from depositories or other sources and are obtained by genetic modification of such Bacillus strains. The industrial Bacillus strains are characterized by being resistant to genetic exchange, such as phage infection or transformation. The strains are stable and may or may not be capable of spore formation. They are usually prototrophic and modified to provide for high yields of endogenous protein products, such as the enzymes alpha-amylase and various proteases. The yield of an endogenous protein product obtained in an industrial production process can amount to at least 5 g/l (0.5% w/v). Industrial strains also secrete DNases, which result in degradation of DNA in the medium, providing for protection against genetic exchange.

Transformation of alkalophilic Bacillus strains will preferably involve the use of protoplasts from said strains. However, the conventional protoplast transformation protocol as described by Cohen et al., supra. does not work for alkalophilic Bacillus strains. Therefore additional protocols had to be developed.

For alkalophilic bacilli, formation and regeneration of protoplasts can occur at high pH, preferably at about pH 8. Protoplasts can be prepared by resuspending the cells in an alkaline holding medium (AHM), pH 7.8 to 8.5, then incubating the cells for 30-80 minutes at 37°C. For an example of an AHM, see Example 5. The resulting protoplasts are then washed to remove lysozyme, then resuspended in AHM. The plasmid construct and the alkalophilic Bacillus host protoplast are then combined in the presence of an appropriate fusogen. While any fusogen may be employed which provides the desired efficiency, for the most part polyethylene glycol (mw 1000-8000) is found to provide high efficiency fusion with great convenience. Protoplasts prepared from the Bacillus acceptor strain are mixed with the plasmid construct for not more than 5 minutes, preferably no longer than 2 minutes in the presence of polyethylene glycol. The fusogen mixture is then replaced with a conventional nutrient medium in which the cells are incubated for up to 5 hours, preferably 2-3 hours, before transfer to regeneration plates. The regeneration plates contain an antibiotic such as neomycin for selection of transformants.

Clones with episomal maintenance of the DNA construct may be identified by detection of expression of the high alkaline serine protease. To identify those clones which contain the expression construct as an integral part of their chromosomes, clones which develop are screened by isolation of total cellular DNA and selection for those clones in which no free plasmid DNA can be detected, but the marker gene of the plasmid is expressed. The clones may then be screened in appropriate ways for detection of the expression of the high alkaline serine protease.

Various detection techniques include use of specific antibodies, DNA or RNA hybridization, formation of enzyme product or disappearance of enzym substrate may be employed for screening the clones to determine the presence of the expression construct and expression of the structural gene of interest, namely protease production. The protease produced can be characterized biochemically by, for example, determination of molecular weight of the protease on SDS-polyacrylamide gel electrophoresis, histidine-MOPS gel electrophoresis and determination of the kinetic parameters Kₘ and Vₘₐₓ, assayed on the synthetic substrate succinyl-L-alanyl-L-alanyl-L-prolyl-L-phenylalanyl-p-nitro-analide.

The Bacillus host containing the expression construct episomally or chromosomally integrated is then grown in a nutrient medium under conditions which favor enzyme synthesis. The nutrient medium usually will include a means of maintaining the plasmid, such as the presence of an antibiotic to which the untransformed host is sensitive. The fermenting may be continued until the broth is exhausted. Where the product has been secreted, the product may be isolated from the broth by conventional techniques, for example by extraction, chromatography, electrophoresis, or the like. Where the product is retained in the cytoplasm, the cells may be harvested by centrifugation, filtration, etc., lysed by mechanical shearing, detergent, lysozyme, or other techniques and the product isolated as described previously. When the untransformed host cell is a high alkaline protease producer, by employing the subject method greatly enhanced yields of high alkaline serine protease can be achieved, usually at least about 120% as compared to the yield of the untransformed host cell, with a single extrachromosomal gene copy.

The following examples are offered by way of illustration and not by way of limitation.

### EXPERIMENTAL

### EXAMPLE 1

### Preparation of a Genomic DNA Library from Alkalophilic Bacillus novo sp. PB92 and Isolation of the Serine Protease Gene

Chromosomal DNA isolated from Bacillus novo sp. PB92 (deposited under No. OR-60 with Laboratorium voor Microbiologie, Technical University of Delft, the Netherlands, see U.S. Patent No. Re. 30,602) according to the procedure described by Saito-Miuva, Biochim. Biophys. Acta 72 (1963) 619-632, was partially digested with the restriction enzyme Sau3A and ligated into the BamHI site of plasmid pUB110 (Gryczan et al., J. Bacteriol. 134 (1978) 318-329). pUB110 plasmid DNA was prepared as described by Birnboim and Doly (Nucl. Acids Res. 7 (1979) 1513-1523).

The ligation mixture was transformed into B. subtilis 1A40 (Bacillus Genetic Stock Centre) according to the method of Spizizen et al., J. Bacteriol. 81 (1961) 741-746, using 0.6-1µg DNA per ml of competent cells. Cells from the transformation mixture were plated on minimal plates containing: 2.8% K₂HPO₄, 1.2% KH₂PO₄, 0.4% (NH₄)₂SO₄, 0.2% tri-Na-citrate.2H₂O, 0.04% MgSO₄.7H₂O, 0.00005% MnSO₄.4H₂O, 0.4% L-glutamic acid, 0.5% glucose, 0.02% casamino acids, 50 µg/ml trypthophan, 20 µg/ml methionine, 20 µg/ml lysine, 20 µg/ml neomycin, 0.4% caseïn and 1.5% agar. After overnight incubation of the plates at 37°C one out of 50,000 neomycin resistant colonies showed increased protease production, as determined by increased precipitation of a halo of casein cleavage products around the colony in the agar plate. Plasmid DNA was isolated from this colony according to the method described by Birnboim and Doly, Nucleic Acids Res. 7 (1979) 1513-1523, and named pM58.

### EXAMPLE 2

### Expression of the PB92 Serine Protease Gene

Bacillus subtilis 1A40 containing pM58 was grown in minimal medium (Spizizen et al., Proc. Natl. Acad. Sci. USA 44 (1958) 1072-1078) to which had been added 0.02% casamino acids 50 µg/ml trypthophan, 20 µg/ml methionine, 20 µg/ml lysine and 20 µg/ml neomycin. After 24 hours, the culture was centrifuged and the supernatant assayed for protease activity using dimethyl casein as substrate (Lin et al., J. Biol. Chem. 244 (1969) 789-793. A culture of B. subtilis 1A40 containing the plasmid pUB110 used as a control showed less than 1/60 of the protease activity shown by the pM58 transformed culture. Protease activity was completely inhibited by treatment with 1 mM phenylsulfonyl fluoride (PMSF), but not by treatment with 20 mM EDTA.

Aliquots of the above described supernatants were analyzed on protein gel according to the method of Laemmli, Nature 227 (1970) 680. Samples for analysis on these gels were prepared by treatment of the supernatants with 5% trichloroacetic acid (TCA). Following centrifugation of the sample the pellet of precipitated protein was washed twice with acetone then dissolved in 40 µl sample buffer (0.5 M Tris/HCl pH 7.5, 10% v/v 2-mercaptoethanol, 50% v/v glycerol and 0.05% Bromophenol Blue) by boiling for 10 minutes. After electrophoresis, the gels were stained using Coomassie Brilliant Blue. Culture supernatant samples were then analyzed by electrophoresis. Three different B. subtilis 1A40 strains were used: a strain containing pUB110; or pM58; or no plasmid; and Bacillus PB92 protease as a control. After electrophoresis, the gels were stained using Commassie Brilliant Blue and destained. The sample from B. subtilis strain 1A40 containing pM58 contained a 31 kD protein, which comigrates with Bacillus PB92 protease. This protein was not detected on the control lane of strain B. subtilis 1A40 containing pUB110.

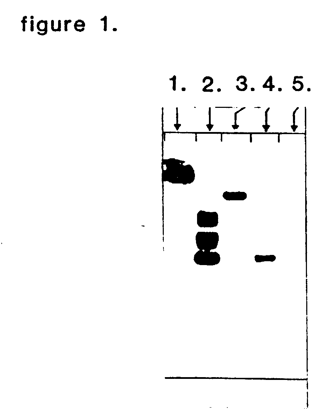

All serine proteases have similar molecular weights. The cloned serine protease of Bacillus PB92 therefore was differentiated from known serine proteases (B. subtilis subtilisin, Carlsberg subtilisin), by transformation of pM58 and pUB110 to the protease negative B. subtilis strain DB104 (R. Doi, J. Bacteriol. 160 (1984) 442-444) and analysis of the extracellular proteases produced. The obtained transformants were grown in minimal medium (Spizizen et al., supra) containing 0.02% casamino acids, 50 µg/ml histidine and 20 µg/ml neomycin. After 24 hours, samples were taken, centrifuged and without pretreatment analysed on histidine/MOPS gels containing 75 mM KOH, 40 mM histidine, 100 mM MOPS (3-(N-morpholino)-propanesulfonic acid), pH 7.5 and 5% polyacrylamide. Electrophoresis buffer contained 40 mM histidine, 100 mM MOPS, pH 6.6. Samples were run in the direction of the cathode. Protease bands were detected with Agfa Pan 100 Professional films (Zuidweg et al., Biotechnol. and Bioengin. 14 (1972) 685-714). These results are shown in Figure 1. As shown, pM58 harbours the gene encoding Bacillus PB92 protease.

### EXAMPLE 3

### Sequencing of the Bacillus PB92 Serine Protease Gene

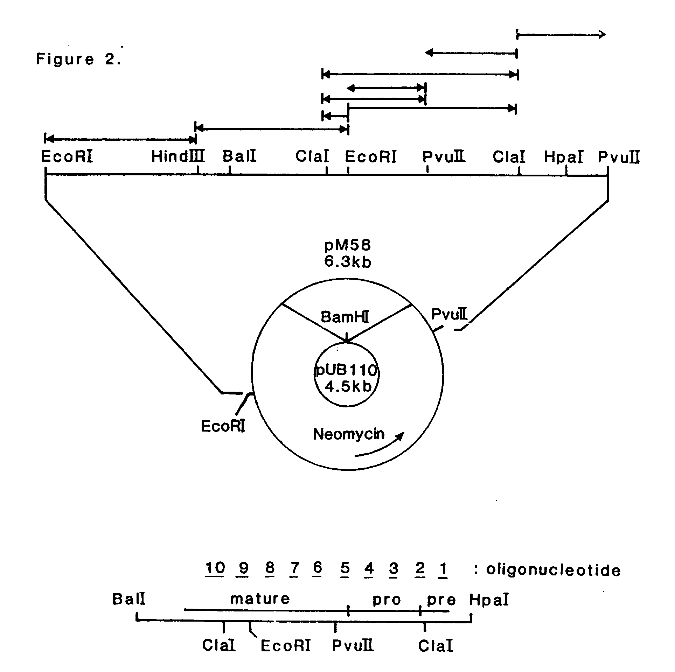

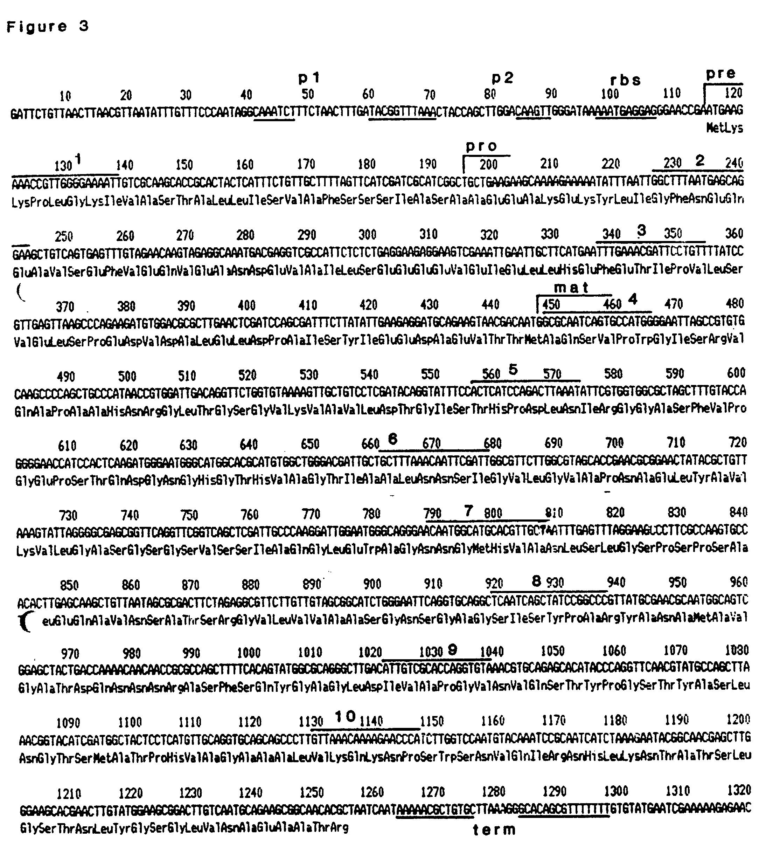

The entire sequence of a BalI-HpaI fragment of pM58 was determined by the method of Sanger, Proc. Natl. Acad. Sci. USA 74 (1977) 6463. Restriction fragments of pM58 (see Figure 2) were cloned in phage M13 vectors mp10, mp11 and mp18 (Messing et al., Nucleic Acids Res. 9 (1981) 309-321. Insertions of pM58 fragments were screened by plaque hybridization. After sequencing, ten oligonucleotides located at regular distances on the gene were made and sequencing was repeated, confirming the sequence shown in Figure 3.

### EXAMPLE 4

### Construction of Serine Protease Containing Plasmid pMAX-4

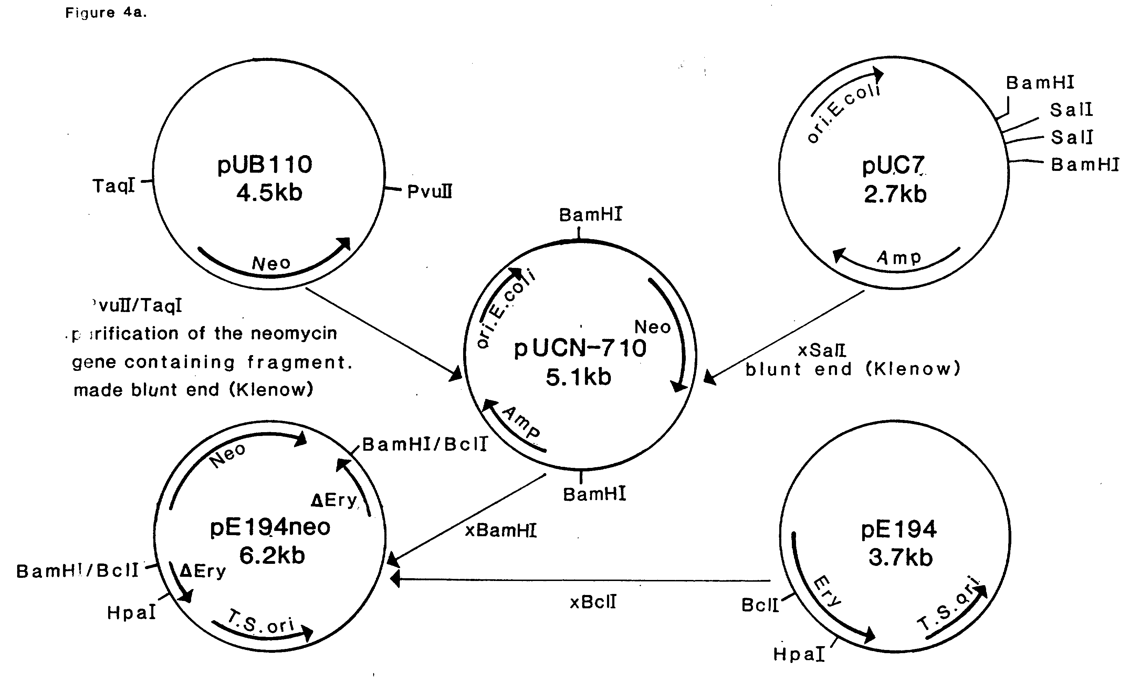

To construct plasmid pUCN710 (Figure 4A) pUB110 was digested with TaqI and PvuII. The fragment containing the gene conferring neomycin resistance was purified on low melting agarose and made blunt with Klenow polymerase and NTP's (Maniatis, Molecular Cloning: A Laboratory Manual. Cold Spring Harbor 1982). Plasmid pUC7 (Vieira et al., Gene 19 (1982) 259-268) was linearized with SalI and made blunt as described above. Both fragments were ligated with T4 ligase (Maniatis) and transformed to E. coli JM103. Selection took place on 2xTY plates (1.6% w/v Bacto-trypton, 1% w/v yeast extract, 0.5% NaCl) containing 50 µg/ml ampicillin and 10 µg/ml neomycin. The resulting plasmid, named pUCN710, was digested with BamHI. The plasmid pE194 (Jordanescu, Plasmid 1 (1978) 468-479) was digested with BclI. The fragments from both digestions were ligated with T4 ligase and transformed to B. subtilis 1A40. Selection took place on minimal plates containing 20 µg/ml neomycin (see Example 1). The plasmid obtained, pE194-neo (Figure 4A) contains the neomycin gene.

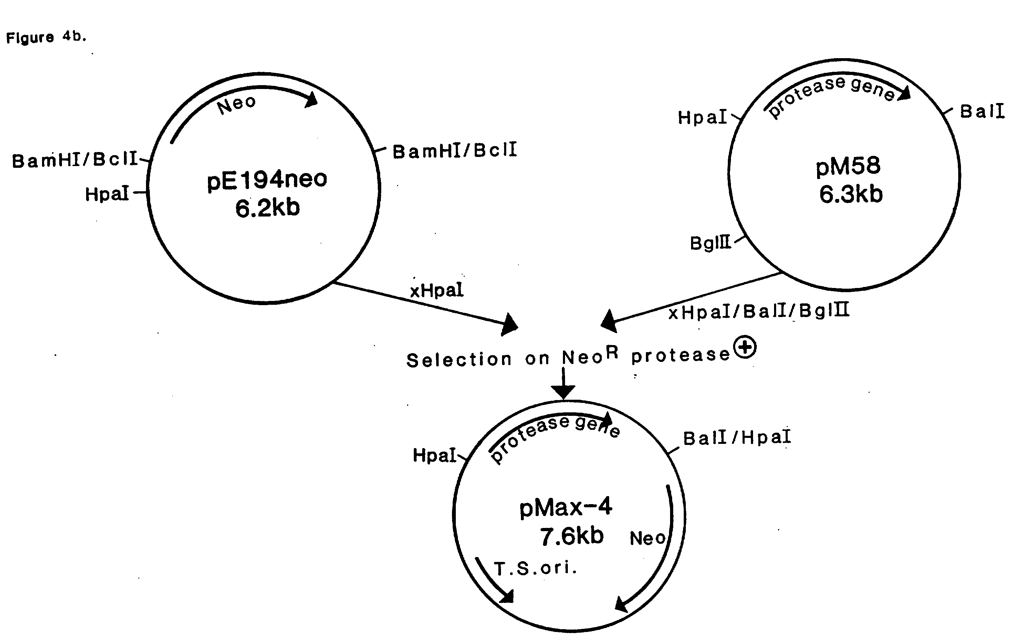

Subcloning of the protease gene in integration vector pE194-neo was performed as follows: pM58 (see Example 1) was digested with HpaI and BalI and BglII. Plasmid pE194-neo was digested with HpaI. These fragments were ligated with T4 ligase and transformed to B. subtilis 1A40. Transformants were selected based upon neomycin resistance and an increase in protease production, as judged by casein cleavage products precipitation (halo formation, see Example 1). Plasmid pMAX-4 was obtained, the structure of which was confirmed by restriction enzyme analysis (see Figure 4B).

### EXAMPLE 5

### Protoplast Transformation of Bacillus Strain PB92 by pMAX-4

Bacillus strain PB92 was grown overnight in 100 ml NBSG-X medium (Thorne et al., J. Bacteriol. 91 (1966) 1012-1020). The culture was centrifuged for 10 minutes at 4,500 rpm in a Sorvall model GSA rotor. Protoplasts were prepared by incubating the bacilli for one hour at 37°C in 10 ml Alkaline Holding Medium (AHM) containing 0.5 M sucrose, 0.02 MgCl₂ and 0.02 M Tris/maleate, pH 8.0, in sterile water to which 0.4 mg/ml lysozyme was added. The protoplasts were pelleted (10 minutes at 4,500 rpm), resuspended in 5 ml AHM⁺ pH 8.0 buffer (AHM buffer to which 3.5% w/v Bacto Penassay Broth and 0.04% w/v Albumine Merieux had been added) mixed, then repelleted as above. After being resuspended in 5.0 ml of alkaline holding medium, 0.5 ml of this suspension of protoplasts were mixed with 5 µg of demineralized water containing 1 µg of plasmid DNA and incubated for 2 minutes in the presence of 30% w/v polyethylene glycol 8,000, pH 8.0. After 1:3 dilution with AHM⁺ pH 8.0 medium and centrifugation, the pellet was resuspended in a small volume (1 ml) of AHM⁺ and incubated for 2-3 hours. One hundred microliter aliquots were plated on freshly prepared regeneration plates containing 0.5 M Na succinate/HCl pH 8.0, 1.5% w/v agar, 0.5% w/v casamino acids, 0.5% w/v yeast extract, 0.031 M phosphate buffer pH 8.0, 0.5% w/v glucose, 0.02 M MgCl₂ and 0.02% w/v Albumine Merieux. These plates also contained 1000 µg/ml neomycin for selection. After incubation at 37°C for at least 72 hrs, the colonies were replica-plated onto heart infusion agar plates containing 20 µg/ml neomycin.

### EXAMPLE 6

### Integration of pMAX-4 in the Bacillus Strain PB92 Chromosome

A colony of Bacillus strain PB92 containing pMAX-4, grown on a Heart Infusion plate containing 20 µg/ml neomycin, was inoculated in 100 ml Tryptone Soya Broth (TSB) containing 1 µg/ml neomycin and incubated for 24 hours at 37°C. Two 2 ml of the culture (approximately 10⁹ cells/ml) was diluted in 100 ml of the same medium, containing 1 µg/ml neomycin, and incubated for 24 hours at 50°C. After 24 hours, 5 ml of the culture (approximately 10⁹ cells/ml) was diluted again, as described above, and incubated for 24 hours at 50°C in the presence of 1 µg/ml neomycin. The last procedure was repeated once more. The cell suspension was then diluted a hundred-fold and plated on Heart Infusion (HI) agar (Difco) plates containing 1 µg/ml neomycin. The plates were incubated for 16 hours at 50°C. Neomycin resistant colonies were isolated and cultured in 10 ml TSB medium containing 1 µg/ml neomycin for 16 h at 37°C. From these cultures, total DNA was isolated (Holmes et al., Anal. Biochem. 114 (1981) 193-197) and checked for plasmid absence by DNA electrophoresis on agarose gel. Samples in which plasmid DNA was not detectable were rechecked by transformation of total DNA to B. subtilis 1A40. Samples lacking the ability to transform B. subtilis 1A40 were considered plasmid free. A neomycin resistant, plasmid free, Bacillus PB92 derived strain was and named PBT109. This strain contained a copy of plasmid pMAX-4 integrated into its chromosome.

Integration in strain PBT109 took place through a so-called Campbell-type mechanism by homologous recombination resulting in two tandemly located protease genes on the chromosome separated by plasmid sequences. This genetic organization of strain PBT109 was confirmed as shown in copending EPA-0 000 000, filed concurrently herewith, the disclosure of which is hereby incorporated by reference.

### EXAMPLE 7

### Protease Production of the Transformed Strains

Protease production of the transformed strains was tested by adding 0.2 ml of a TSB culture containing approximately 10⁹ cells/ml to 500 ml shake flasks containing 100ml of the production medium described in U.S. Patent No. Re. 30,602. When B. subtilis strains were tested, however, the pH was adjusted to pH 7.0. Neomycin was added at a concentration of 20 µg/ml for the strains DB104 (pUB 110), DB104 (pMAX-4), PB92 (pMAX-4) and at a concentration of 1 µg/ml for PBT109. The relative protease production for these cell lines is shown in Table 1.

**Table 1**

| Production of Protease in Transformed Bacilli | | |
|---|---|---|
| Strain | Relative Protease Production** | Neomycin Addition |
| Bacillus PB92 | 100.0% | - |
| Bacillus PB92 (pMAX-4) | 120.0% | + |
| Bacillus PBT109 | 120.0% | + |
| B. subtilis DB104 (pUB110)* | 1.5% | + |
| B. subtilis DB104 (pMAX-4) | 10.0% | + |

| | | |
|---|---|---|
| * To measure protease production from the protease gene of pMAX-4 only, this plasmid was also transformed (Spizizen et al., (1961) supra) to the exoprotease negative B. subtilis strain DB104 (R. Doi, J. Bacteriol. 160 (1984) 442-444), as described in Example 2. | | |
| **Protease activity was assayed using dimethyl casein as substrate as described by Lin et al. J. Biol. Chem. 244 (1969) 789-793. | | |

In fermentations at a larger scale (10 liters), Bacillus PB92 (pMAX-4) showed instability and a decrease in production as compared with PBT109, if no neomycin was added to the fermentation medium.

It is evident from the above results that a simple and efficient procedure is provided for production of serine protease by stably introducing a homologous or heterologous gene encoding serine protease into the chromosome of industrial Bacillus strains. Enhanced production of a serine protease in a Bacillus host which already produces a serine protease is also provided. Integration of plasmid constructs into the host cell chromosomes results in a more stable situation for production fermentations and for a greatly enhanced protease production than when extrachromosomal plasmid constructs are present.

All publication and patent appliations mentioned in this specification are indicative of the level of skill of those skilled in the art to which this invention pertains.

The invention now being fully described, it will be apparent to one of ordinary skill in the art that many changes and modifications can be made thereto within the scope of the appended claims.

## Claims (Claims for the following Contracting State(s): AT, BE, DE, FR, GB, IT, NL)

1. An expression cassette comprising:
as operably joined components, (a) a transcriptional regulatory region and a translational initiation region functional in a Bacillus host cell, (b) a DNA sequence encoding a high alkaline serine protease, whose sequence has at least 70% homology to: 
H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH,
 (c) a translational and transcriptional termination region functional in said host cell, wherein expression of said DNA sequence is regulated by said initiation and termination regions; and (d) at least one of a marker gene and a temperature-sensitive origin of replication of a bacterial plasmid, whereby the high alkaline protease has an optimum activity at pH value higher than 9 and retain at least 80% of the optimal activity at a pH of 11 or higher and is obtainable from an alkalopilic Bacillus strain.

2. An expression cassette according Claim 1, wherein said protease is obtainable from Bacillus novo species PB92.

3. An expression cassette according to Claim 1, wherein said DNA sequence encodes a polypeptide comprising substantially the following amino acid sequence:

H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH.

4. A transformed Bacillus host cell capable of producing high-alkaline serine protease comprising the expression cassette of claim 1, 2 or 3.

5. A transformed Bacillus host according to Claim 4, wherein said cassette is stably incorporated into the host chromosome.

6. A transformed Bacillus host cell according to Claim 4 or 5, comprising at least two copies of said DNA sequence integrated in the chromosome of said cell.

7. A transformed Bacillus host cell according to Claim 4, wherein an untransformed parent of said host cell is a wild-type Bacillus which produces a serine protease.

8. A transformed Bacillus host cell according to Claim 4, wherein an untransformed parent of said host cell is a mutant strain of a Bacillus which produces a serine protease.

9. A transformed Bacillus host cell according to Claim 4, wherein an untransformed parent of said host cell is a mutant strain of a Bacillus which does not produce a serine protease.

10. A transformed Bacillus host cell according to any one of Claims 4 to 9, wherein said high alkaline serine protease is obtainable from Bacillus novo species PB92.

11. A transformed Bacillus host cell according to any one of Claims 4 to 10, wherein said DNA sequence encodes substantially the following amino acid sequence:

H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH.

12. A method for transforming Bacillus host cells which comprises:
providing the expression cassette of any one of claims 1 to 3 encoding a high alkaline protease;
combining a protoplast prepared from a Bacillus host cell with said cassette in the presence of a fusogen at alkaline pH, whereby said cassette is introduced into said host cell and nucleic acid encoding said protease is integrated into the chromosome of said host cell; and
selecting host cells stably containing said protease gene and which are capable of producing high alkaline protease, whereby the high alkaline protease has an optimum activity at pH value higher than 9 and retain at least 80% of the optimal activity at a pH of 11 or higher and is obtainable from an alkalopilic Bacillus strain.

13. A method according to Claim 12, wherein said fusogen is polyethylene glycol at a concentration of about 30% w/v.

14. A method according to Claims 12 or 13, wherein said polypeptide of interest comprises essentially the following amino acid sequences:

H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH.

15. Use of a transformed Bacillus host as defined in any of Claims 4 to 11 or as obtained by a method as defined in any one of Claims 12 to 13, for the production of a high alkaline serine protease.

16. A method for producing a high alkaline serine protease in Bacillus, said method comprising:
growing a Bacillus culture comprising the Bacillus cells of any one of claims 4 to 11.

17. A method according to Claim 16, wherein said plasmid construct further comprises:
a second DNA sequence encoding a secretory leader and a processing signal operably joined 5' to said first DNA sequence, whereby said expression product is secreted.

18. A method according to Claims 16 or 17 further comprising:
isolating said expression product.

## Claims (Claims for the following Contracting State(s): ES)

1. A method for transforming Bacillus host cells which comprises:
providing an expression cassette comprising
as operably joined components, (a) a transcriptional regulatory region and a translational initiation region functional in a Bacillus host cell, (b) a DNA sequence encoding a high alkaline serine protease, whose sequence has at least 70% homology to: 
H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH,
 (c) a translational and transcriptional termination region functional in said host cell, wherein expression of said DNA sequence is regulated by said initiation and termination regions; and (d) at least one of a marker gene and a temperature-sensitive origin of replication of a bacterial plasmid, whereby the high alkaline protease has an optimum activity at pH value higher than 9 and retain at least 80% of the optimal activity at a pH of 11 or higher and is obtainable from an alkalopilic Bacillus strain;
combining a protoplast prepared from a Bacillus host cell with said cassette in the presence of a fusogen at alkaline pH, whereby said cassette is introduced into said host cell and nucleic acid encoding said protease is integrated into the chromosome of said host cell; and
selecting host cells stably containing said protease gene and which are capable of producing high alkaline protease, whereby the high alkaline protease has an optimum activity at pH value higher than 9 and retain at least 80% of the optimal activity at a pH of 11 or higher and is obtainable from an alkalopilic Bacillus strain.

2. A method according Claim 1, wherein said protease is obtainable from Bacillus novo species PB92.

3. A method according to Claim 1 or 2, wherein said fusogen is polyethylene glycol at a concentration of about 30% w/v.

4. A method according to Claims 1, 2 or 3, wherein said polypeptide of interest comprises essentially the following amino acid sequences:

H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH.

5. Use of a transformed Bacillus host as obtained by a method as defined in any one of Claims 1 to 4, for the production of a high alkaline serine protease.

6. A method for producing a high alkaline serine protease in Bacillus, said method comprising:
growing a Bacillus culture comprising the transferred Bacillus host cells comprising the expression cassette defined in any one of claims 1 to 4.

7. A method according to Claim 6, wherein said plasmid construct further comprises:
a second DNA sequence encoding a secretory leader and a processing signal operably joined 5' to said first DNA sequence, whereby said expression product is secreted.

8. A method according to Claims 6 or 7 further comprising:
isolating said expression product.

## Patentansprüche (Patentansprüche für folgende(n) Vertragsstaat(en): AT, BE, DE, FR, GB, IT, NL)

1. Expressionskassette, umfassend:
als operabel oder funktional verknüpfte Komponenten (a) einen Transkriptionsregulationsbereich und einen Translationsinitiationsbereich, die in einer Bacillus-Wirtszelle funktional sind,
(b) eine DNA-Sequenz, die eine hochalkalische Serinprotease kodiert, deren Sequenz mindestens 70% Homologie aufweist zu: 
H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH,
 (c) einen Translations- und Transkriptionsterminationsbereich, der in der Wirtszelle funktional ist, wobei eine Expression der DNA-Sequenz durch die Initiations- und Terminationsbereiche reguliert wird, und (d) mindestens eines von einem Markergen und einem temperaturempfindlichen Replikationsursprung eines bakteriellen Plasmids, wobei die hochalkalische Protease eine optimale Aktivität bei einem pH-Wert höher als 9 aufweist und mindestens 80% der optimalen Aktivität bei einem pH von 11 oder höher bewahrt und aus einem alkalophilen Bacillus-Stamm erhalten werden kann.

2. Expressionskassette nach Anspruch 1, wobei die Protease aus Bacillus novo species PB92 erhalten werden kann.

3. Expressionskassette nach Anspruch 1, wobei die DNA-Sequenz ein Polypeptid kodiert, das im wesentlichen die folgende Aminosäuresequenz umfaßt:

H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH.

4. Transformierte Bacillus-Wirtszelle, die in der Lage ist, hochalkalische Serinprotease zu produzieren, umfassend die Expressionskassette von Anspruch 1, 2 oder 3.

5. Transformierter Bacillus-Wirt nach Anspruch 4, wobei die Kassette stabil in das Wirtschromosom integriert ist.

6. Transformierte Bacillus-Wirtszelle nach Anspruch 4 oder 5, umfassend mindestens zwei Kopien der DNA-Sequenz, die in das Chromosom der Zelle integriert sind.

7. Transformierte Bacillus-Wirtszelle nach Anspruch 4, wobei eine nicht-transformierte Elternzelle der Wirtszelle ein Wildtyp-Bacillus ist, der eine Serinprotease produziert.

8. Transformierte Bacillus-Wirtszelle nach Anspruch 4, wobei eine nicht-transformierte Elternzelle der Wirtszelle ein mutierter Stamm eines Bacillus ist, der eine Serinprotease produziert.

9. Transformierte Bacillus-Wirtszelle nach Anspruch 4, wobei eine nicht-transformierte Elternzelle der Wirtszelle ein mutierter Stamm eines Bacillus ist, der keine Serinprotease produziert.

10. Transformierte Bacillus-Wirtszelle nach einem der Ansprüche 4 bis 9, wobei die hochalkalische Serinprotease aus Bacillus novo species PB92 erhalten werden kann.

11. Transformierte Bacillus-Wirtszelle nach einem der Ansprüche 4 bis 10, wobei die DNA-Sequenz im wesentlichen die folgende Aminosäuresequenz kodiert:

H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH.

12. Verfahren zum Transformieren von Bacillus-Wirtszellen, welches umfaßt:
eine Expressionskassette nach einem der Ansprüche 1 bis 3, die eine hochalkalische Protease kodiert, bereitzustellen,
einen aus einer Bacillus-Wirtszelle hergestellten Protoplasten mit der Kassette zusammenzugeben in Gegenwart eines Fusogens bei alkalischem pH, wodurch die Kassette in die Wirtszelle eingeschleust wird und die genannte Protease kodierende Nukleinsäure in das Chromosom der Wirtszelle integriert wird, und
Wirtszellen zu selektieren, die das Proteasegen stabil enthalten und in der Lage sind, hochalkalische Protease zu produzieren, wobei die hochalkalische Protease eine optimale Aktivität bei einem pH-Wert höher als 9 aufweist und mindestens 80% der optimalen Aktivität bei einem pH von 11 oder höher bewahrt und aus einem alkalophilen Bacillus-Stamm erhalten werden kann.

13. Verfahren nach Anspruch 12, wobei das Fusogen Polyethylenglycol in einer Konzentration von ungefähr 30% (Gew./Vol.) ist.

14. Verfahren nach den Ansprüchen 12 oder 13, wobei das Polypeptid von Interesse im wesentlichen die folgenden Aminosäuresequenzen umfaßt:

H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH.

15. Verwendung eines transformierten Bacillus-Wirts, wie in einem der Ansprüche 4 bis 11 definiert oder erhalten durch ein Verfahren, wie in einem der Ansprüche 12 bis 13 definiert, für die Produktion einer hochalkalischen Serinprotease.

16. Verfahren zum Herstellen einer hochalkalischen Serinprotease in Bacillus, wobei das Verfahren umfaßt:
eine Bacillus-Kultur, umfassend die Bacillus-Zellen nach einem der Ansprüche 4 bis 11, zu züchten.

17. Verfahren nach Anspruch 16, wobei das Plasmidkonstrukt ferner umfaßt:
eine zweite DNA-Sequenz, die eine sekretorische Leadersequenz und ein Prozessierungssignal, die operabel oder funktional 5' mit der ersten DNA-Sequenz verknüpft sind, kodiert, wodurch das Expressionsprodukt sezerniert wird.

18. Verfahren nach den Ansprüchen 16 oder 17, welches ferner umfaßt:
das Expressionsprodukt zu isolieren.

## Patentansprüche (Patentansprüche für folgende(n) Vertragsstaat(en): ES)

1. Verfahren zum Transformieren von Bacillus-Wirtszellen, welches umfaßt:
eine Expressionskassette bereitzustellen, umfassend als operabel oder funktional verknüpfte Komponenten
(a) einen Transkriptionsregulationsbereich und einen Translationsinitiationsbereich, die in einer Bacillus-Wirtszelle funktional sind,
(b) eine DNA-Sequenz, die eine hochalkalische Serinprotease kodiert, deren Sequenz mindestens 70% Homologie aufweist zu: 
H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH,
(c) einen Translations- und Transkriptionsterminationsbereich, der in der Wirtszelle funktional ist, wobei eine Expression der DNA-Sequenz durch die Initiations- und Terminationsbereiche reguliert wird, und (d) mindestens eines von einem Markergen und einem temperaturempfindlichen Replikationsursprung eines bakteriellen Plasmids, wobei die hochalkalische Protease eine optimale Aktivität bei einem pH-Wert höher als 9 aufweist und mindestens 80% der optimalen Aktivität bei einem pH von 11 oder höher bewahrt und aus einem alkalophilen Bacillus-Stamm erhalten werden kann;
einen aus einer Bacillus-Wirtszelle hergestellten Protoplasten mit der Kassette zusammenzugeben in Gegenwart eines Fusogens bei alkalischem pH, wodurch die Kassette in die Wirtszelle eingeschleust wird und die genannte Protease kodierende Nukleinsäure in das Chromosom der Wirtszelle integriert wird, und
Wirtszellen zu selektieren, die das Proteasegen stabil enthalten und in der Lage sind, hochalkalische Protease zu produzieren, wobei die hochalkalische Protease eine optimale Aktivität bei einem pH-Wert höher als 9 aufweist und mindestens 80% der optimalen Aktivität bei einem pH von 11 oder höher bewahrt und aus einem alkalophilen Bacillus-Stamm erhalten werden kann.

2. Verfahren nach Anspruch 1, wobei die Protease aus Bacillus novo species PB92 erhalten werden kann.

3. Verfahren nach Anspruch 1 oder 2, wobei das Fusogen Polyethylenglycol in einer Konzentration von ungefähr 30% (Gew./Vol.) ist.

4. Verfahren nach den Ansprüchen 1, 2 oder 3, wobei das Polypeptid von Interesse im wesentlichen die folgenden Aminosäuresequenzen umfaßt:

H2N-A-Q-S-V-P-W-G-I-S-R-V-Q-A-P-A-A-H-N-R-G-L-T-G-S-G-V-K-V-A-
V-L-D-T-G-I-S-T-H-P-D-L-N-I-R-G-G-A-S-F-V-P-G-E-P-S-T-Q-D-G-N-
G-H-G-T-H-V-A-G-T-I-A-A-L-N-N-S-I-G-V-L-G-V-A-P-N-A-E-L-Y-A-V-
K-V-L-G-A-S-G-S-G-S-V-S-S-I-A-Q-G-L-E-W-A-G-N-N-G-M-H-V-A-N-L-
S-L-G-S-P-S-P-S-A-T-L-E-Q-A-V-N-S-A-T-S-R-G-V-L-V-V-A-A-S-G-N-
S-G-A-G-S-I-S-Y-P-A-R-Y-A-N-A-M-A-V-G-A-T-D-Q-N-N-N-R-A-S-F-S-
Q-Y-G-A-G-L-D-I-V-A-P-G-V-N-V-Q-S-T-Y-P-G-S-T-Y-A-S-L-N-G-T-S-
M-A-T-P-H-V-A-G-A-A-A-L-V-K-Q-K-N-P-S-W-S-N-V-Q-I-R-N-H-L-K-N-
T-A-T-S-L-G-S-T-N-L-Y-G-S-G-L-V-N-A-E-A-A-T-R-COOH.

5. Verwendung eines transformierten Bacillus-Wirts, wie erhalten durch ein Verfahren, wie in einem der Ansprüche 1 bis 4 definiert, für die Produktion einer hochalkalischen Serinprotease.

6. Verfahren zum Herstellen einer hochalkalischen Serinprotease in Bacillus, wobei das Verfahren umfaßt:
eine Bacillus-Kultur, umfassend die transformierten Bacillus-Wirtszellen, die die in einem der Ansprüche 1 bis 4 definierte Expressionskassette umfassen, zu züchten.

7. Verfahren nach Anspruch 6, wobei das Plasmidkonstrukt ferner umfaßt:
eine zweite DNA-Sequenz, die eine sekretorische Leadersequenz und ein Prozessierungssignal, die operabel oder funktional 5' mit der ersten DNA-Sequenz verknüpft sind, kodiert, wodurch das Expressionsprodukt sezerniert wird.

8. Verfahren nach Anspruch 6 oder 7, welches ferner umfaßt: das Expressionsprodukt zu isolieren.

## Revendications (Revendications pour l'(les) Etat(s) contractant(s) suivant(s): AT, BE, DE, FR, GB, IT, NL)

1. Cassette d'expression comprenant :
en tant que composants liés de manière opérationnelle, (a) une région régulant la transcription et une région d'initiation de la traduction fonctionnelles dans une cellule hôte de *Bacillus ;* (b) une séquence d'ADN codant pour une sérine protéase hautement alcaline, dont la séquence présente une homologie d'au moins 70% avec : 
H2N-A-Q-S-V-P-W-G-I-S-R-V-Q-A-P-A-A-H-N-R-G-L-T-G-S-G-V-
K-V-A-V-L-D-T-G-I-S-T-H-P-D-L-N-I-R-G-G-A-S-F-V-P-G-E-P-
S-T-Q-D-G-N-G-H-G-T-H-V-A-G-T-I-A-A-L-N-N-S-I-G-V-L-G-V-
A-P-N-A-E-L-Y-A-V-K-V-L-G-A-S-G-S-G-S-V-S-S-I-A-Q-G-L-E-
W-A-G-N-N-G-M-H-V-A-N-L-S-L-G-S-P-S-P-S-A-T-L-E-Q-A-V-N-
S-A-T-S-R-G-V-L-V-V-A-A-S-G-N-S-G-A-G-S-I-S-Y-P-A-R-Y-A-
N-A-M-A-V-G-A-T-D-Q-N-N-N-R-A-S-F-S-Q-Y-G-A-G-L-D-I-V-A-
P-G-V-N-V-Q-S-T-Y-P-G-S-T-Y-A-S-L-N-G-T-S-M-A-T-P-H-V-A-
G-A-A-A-L-V-K-Q-K-N-P-S-W-S-N-V-Q-I-R-N-H-L-K-N-T-A-T-S-
L-G-S-T-N-L-Y-G-S-G-L-V-N-A-E-A-A-T-R-COOH,
 (c) une région de terminaison de la transcription et de la traduction fonctionnelle dans ladite cellule hôte, dans laquelle l'expression de ladite séquence d'ADN est régulée par lesdites régions d'initiation et de terminaison ; et (d) au moins un gène marqueur ou une origine de réplication sensible à la température provenant d'un plasmide bactérien, grâce à quoi la protéase hautement alcaline présente une activité optimale à une valeur de pH supérieure à 9 et conserve au moins 80% de l'activité optimale à un pH égal ou supérieur à 11 et peut être obtenu à partir d'une souche de *Bacillus* alcalophile.

2. Cassette d'expression selon la revendication 1, dans laquelle ladite protéase peut être obtenue à partir de *Bacillus* novo species PB92.

3. Cassette d'expression selon la revendication 1, dans laquelle la séquence d'ADN code pour un polypeptide comprenant sensiblement la séquence d'acides aminés suivante :

H2N-A-Q-S-V-P-W-G-I-S-R-V-Q-A-P-A-A-H-N-R-G-L-T-G-S-G-V-
K-V-A-V-L-D-T-G-I-S-T-H-P-D-L-N-I-R-G-G-A-S-F-V-P-G-E-P-
S-T-Q-D-G-N-G-H-G-T-H-V-A-G-T-I-A-A-L-N-N-S-I-G-V-L-G-V-
A-P-N-A-E-L-Y-A-V-K-V-L-G-A-S-G-S-G-S-V-S-S-I-A-Q-G-L-E-
W-A-G-N-N-G-M-H-V-A-N-L-S-L-G-S-P-S-P-S-A-T-L-E-Q-A-V-N-
S-A-T-S-R-G-V-L-V-V-A-A-S-G-N-S-G-A-G-S-I-S-Y-P-A-R-Y-A-
N-A-M-A-V-G-A-T-D-Q-N-N-N-R-A-S-F-S-Q-Y-G-A-G-L-D-I-V-A-
P-G-V-N-V-Q-S-T-Y-P-G-S-T-Y-A-S-L-N-G-T-S-M-A-T-P-H-V-A-
G-A-A-A-L-V-K-Q-K-N-P-S-W-S-N-V-Q-I-R-N-H-L-K-N-T-A-T-S-
L-G-S-T-N-L-Y-G-S-G-L-V-N-A-E-A-A-T-R-COOH.

4. Cellule hôte de *Bacillus* transformée capable de produire de la sérine protéase hautement alcaline comprenant la cassette d'expression selon la revendication 1, 2 ou 3.

5. Cellule hôte de *Bacillus* transformée selon la revendication 4, dans laquelle ladite cassette d'expression est incorporée de façon stable dans le chromosome de l'hôte.

6. Cellule hôte de *Bacillus* transformée selon la revendication 4 ou 5, comprenant au moins deux copies de ladite séquence d'ADN intégrées dans le chromosome de ladite cellule.

7. Cellule hôte de *Bacillus* transformée selon la revendication 4, dans laquelle un parent non transformé de ladite cellule hôte est un *Bacillus* de type sauvage qui produit une sérine protéase.

8. Cellule hôte de *Bacillus* transformée selon la revendication 4, dans laquelle un parent non transformé de ladite cellule hôte est une souche mutante d'un *Bacillus* qui produit une sérine protéase.

9. Cellule hôte de *Bacillus* transformée selon la revendication 4, dans laquelle un parent non transformé de ladite cellule hôte est une souche mutante d'un *Bacillus* qui ne produit pas une sérine protéase.

10. Cellule hôte de *Bacillus* transformée selon l'une quelconque des revendication 4 à 9, dans laquelle ladite sérine protéase hautement alcaline peut être obtenue à partir de *Bacillus* novo species PB92.

11. Cellule hôte de *Bacillus* transformée selon l'une quelconque des revendication 4 à 10, dans laquelle ladite séquence d'ADN code sensiblement pour la séquence d'acides aminés suivante :

H2N-A-Q-S-V-P-W-G-I-S-R-V-Q-A-P-A-A-H-N-R-G-L-T-G-S-G-V-
K-V-A-V-L-D-T-G-I-S-T-H-P-D-L-N-I-R-G-G-A-S-F-V-P-G-E-P-
S-T-Q-D-G-N-G-H-G-T-H-V-A-G-T-I-A-A-L-N-N-S-I-G-V-L-G-V-
A-P-N-A-E-L-Y-A-V-K-V-L-G-A-S-G-S-G-S-V-S-S-I-A-Q-G-L-E-
W-A-G-N-N-G-M-H-V-A-N-L-S-L-G-S-P-S-P-S-A-T-L-E-Q-A-V-N-
S-A-T-S-R-G-V-L-V-V-A-A-S-G-N-S-G-A-G-S-I-S-Y-P-A-R-Y-A-
N-A-M-A-V-G-A-T-D-Q-N-N-R-A-S-F-S-Q-Y-G-A-G-L-D-I-V-A-
P-G-V-N-V-Q-S-T-Y-P-G-S-T-Y-A-S-L-N-G-T-S-M-A-T-P-H-V-A-
G-A-A-A-L-V-K-Q-K-N-P-S-W-S-N-V-Q-I-R-N-H-L-K-N-T-A-T-S-
L-G-S-T-N-L-Y-G-S-G-L-V-N-A-E-A-A-T-R-COOH.

12. Procédé pour transformer des cellules hôtes de *Bacillus* qui comprend :
la fourniture de la cassette d'expression selon l'une quelconque des revendications 1 à 3 codant pour une protéase hautement alcaline ;
la combinaison d'un protoplaste préparé à partir d'une cellule hôte de *Bacillus* avec ladite cassette en présence d'un fusogène, à un pH alcalin, grâce à quoi la cassette est introduite dans ladite cellule hôte et l'acide nucléique codant pour ladite protéase est intégré dans le chromosome de ladite cellule hôte ;
la sélection des cellules hôtes contenant de façon stable ledit gène de la protéase et qui sont capables de produire la protéase hautement alcaline, grâce à quoi la protéase hautement alcaline présente une activité optimale à une valeur de pH supérieure à 9 et conserve au moins 80% de son activité optimale à une valeur de pH égale ou supérieure à 11 et peut être obtenue à partir d'une souche de *Bacillus* alcalophile.

13. Procédé selon la revendication 12, dans lequel ledit fusogène est le polyéthylène glycol à une concentration d'environ 30% en poids/volume.

14. Procédé selon la revendication 12 ou 13, dans lequel ledit polypeptide d'intérêt comprend sensiblement la séquence d'acides aminés suivante :

H2N-A-Q-S-V-P-W-G-I-S-R-V-Q-A-A-H-N-R-G-L-T-G-S-G-V-K-V-
A-V-L-D-T-G-I-S-T-H-P-D-L-N-I-R-G-G-A-S-F-V-P-G-E-P-S-T-
Q-D-G-N-G-H-G-T-H-V-A-G-T-I-A-A-L-N-N-S-I-G-V-L-G-V-A-P-
N-A-E-L-Y-A-V-K-V-L-G-A-S-G-S-G-S-V-S-S-I-A-Q-G-L-E-W-A-
G-N-N-G-M-H-V-A-N-L-S-L-G-S-P-S-P-S-A-T-L-E-Q-A-V-N-S-A-
T-S-R-G-V-L-V-V-A-A-S-G-N-S-G-A-G-S-I-S-Y-P-A-R-Y-A-N-A-
M-A-V-G-A-T-D-Q-N-N-N-R-A-S-F-S-Q-Y-G-A-G-L-D-I-V-A-P-G-
V-N-V-Q-S-T-Y-P-G-S-T-Y-P-G-S-T-Y-A-S-L-N-G-T-S-M-A-T-P-
H-V-A-G-A-A-A-L-V-K-Q-K-N-P-S-W-S-N-V-Q-I-R-N-H-L-K-N-T-
A-T-S-L-G-S-T-N-L-Y-G-S-G-L-V-N-A-E-A-A-T-R-COOH.

15. Utilisation d'un *Bacillus* hôte transformé tel que défini dans l'une quelconque des revendications 4 à 11 ou tel qu'il est obtenu selon un procédé défini dans l'une quelconque des revendications 12 à 13, pour la production d'une sérine protéase hautement alcaline.

16. Procédé de production d'une sérine protéase hautement alcaline dans *Bacillus,* ledit procédé comprenant :
la mise en culture de cellules de *Bacillus* selon l'une quelconque des revendications 4 à 11.

17. Procédé selon la revendication 16, dans lequel ledit produit de construction plasmidique comprend en outre :
une seconde séquence d'ADN codant pour une séquence leader sécrétoire et une séquence signal de traitement liés de manière opérationnelle dans le sens 5' par rapport à ladite première séquence d'ADN, par laquelle le produit d'expression est sécrété.

18. Procédé selon la revendication 16 ou 17 comprenant en outre :
l'isolement dudit produit d'expression.

## Revendications (Revendications pour l'(les) Etat(s) contractant(s) suivant(s): ES)

1. Procédé de transformation de cellules hôtes de *Bacillus* qui comprend:
la fourniture d'une cassette d'expression comprenant :
en tant que composants liés de manière opérationnelle, (a) une région régulant la transcription et une région d'initiation de la traduction fonctionnelles dans une cellule hôte de Bacillus, (b) une séquence d'ADN codant pour une sérine protéase alcaline, dont la séquence présente une homologie d'au moins 70% avec : 
H2N-A-Q-S-V-P-W-G-I-S-R-V-Q-A-P-A-A-H-N-R-G-L-T-G-S-G-V-K-V-A-V-L-D-
T-G-I-S-T-H-P-D-L-N-I-R-G-G-A-S-F-V-P-G-E-P-S-T-Q-D-G-N-G-H-G-T-H-V-A-
G-T-I-A-A-L-N-N-S-I-G-V-L-G-V-A-P-N-A-E-L-Y-A-V-K-V-L-G-A-S-G-S-G-S-V-
S-S-I-A-Q-G-L-E-W-A-G-N-N-G-M-H-V-A-N-L-S-L-G-S-P-S-P-S-A-T-L-E-Q-A-V-
N-S-A-T-S-R-G-V-L-V-V-A-A-S-G-N-S-G-A-G-S-I-S-Y-P-A-R-Y-A-N-A-M-A-V-
G-A-T-D-Q-N-N-N-R-A-S-F-S-Q-Y-G-A-G-L-D-I-V-A-P-G-V-N-V-Q-S-T-Y-P-G-
S-T-Y-A-S-L-N-G-T-S-M-A-T-P-H-V-A-G-A-A-A-L-V-K-Q-K-N-P-S-W-S-N-V-Q-
I-R-N-H-L-K-N-T-A-T-S-L-G-S-T-N-L-Y-G-S-G-L-V-N-A-E-A-A-T-R-COOH,
 (c) une région de terminaison de la traduction et de la transcription fonctionnelle dans ladite cellule hôte, dans laquelle l'expression de ladite séquence d'ADN est régulée par lesdites régions d'initiation et de terminaison ; et (d) au moins un gène marqueur ou une origine de réplication sensible à la température provenant d'un plasmide bactérien, grâce à quoi la protéase hautement alcaline présente une activité optimale à une valeur de pH supérieure à 9 et conserve au moins 80% de l'activité optimale à un pH égal ou supérieur à 11 et peut être obtenue à partir d'une souche de *Bacillus* alcalophile ;
la combinaison d'un protoplaste préparé à partir d'une cellule hôte de *Bacillus* avec ladite cassette en présence d'un fusogène à un pH alcalin, grâce à quoi ladite cassette est introduite dans ladite cellule hôte et l'acide nucléique codant pour ladite protéase est incorporé dans le chromosome de ladite cellule hôte ; et
la sélection de cellules *hôtes qui* contiennent de manière stable ledit gène de la protéase et qui sont capables de produire la protéase hautement alcaline, grâce à quoi la protéase hautement alcaline présente une activité optimale à une valeur de pH supérieure à 9 et conserve au moins 80% de son activité optimale à un pH égal ou supérieur à 11 et peut être obtenue à partir d'une souche de *Bacillus* alcalophile.

2. Procédé selon la revendication 1, dans lequel ladite protéase peut être obtenue à partir de *Bacillus* novo species PB92.

3. Procédé selon la revendication 1 ou 2, dans lequel ledit fusogène est le polyéthylène glycol à une concentration d'environ 30% en poids/volume.

4. Procédé selon la revendication 1, 2 ou 3, dans lequel ledit polypeptide d'intérêt comprend sensiblement la séquence d'acides aminés suivante:

H2N-A-Q-S-V-P-W-G-I-S-R-V-Q-A-P-A-A-H-N-R-G-L-T-G-S-G-V-K-V-A-V-L-D-
T-G-I-S-T-H-P-D-L-N-I-R-G-G-A-S-F-V-P-G-E-P-S-T-Q-D-G-N-G-H-G-T-H-V-A-
G-T-I-A-A-L-N-N-S-I-G-V-L-G-V-A-P-N-A-E-L-Y-A-V-K-V-L-G-A-S-G-S-G-V-S-
S-I-A-Q-G-L-E-W-A-G-N-N-G-M-H-V-A-N-L-S-L-G-S-P-S-P-S-A-T-L-E-Q-A-V-
N-S-A-T-S-R-G-V-L-V-V-A-A-S-G-N-S-G-A-G-S-I-S-Y-P-A-R-Y-A-N-A-M-A-V-
G-A-T-D-Q-N-N-N-R-A-S-F-S-Q-Y-G-A-G-L-D-I-V-A-P-G-V-N-V-Q-S-T-Y-P-G-

S-T-Y-A-S-L-N-G-T-S-M-A-T-P-H-V-A-G-A-A-A-L-V-K-Q-K-N-P-S-W-S-N-V-Q-
I-R-N-H-L-K-N-T-A-T-S-L-G-S-T-N-L-Y-G-S-G-L-V-N-A-E-A-A-T-R-COOH.

5. Utilisation d'un hôte de *Bacillus* transformé tel qu'il est obtenu par un procédé défini dans l'une quelconque des revendications 1 à 4, pour la production d'une sérine protéase hautement alcaline.

6. Procédé de production d'une sérine protéase hautement alcaline dans un *Bacillus,* ledit procédé comprenant :
la mise en culture de cellules de *Bacillus* comprenant les cellules hôtes de *Bacillus* transformées comprenant la cassette d'expression définie dans l'une quelconque des revendications 1 à 4.

7. Procédé selon la revendication 6, dans lequel ledit produit de construction plasmidique comprend en outre :
une seconde séquence d'ADN codant pour une séquence leader sécrétoire et une séquence signal de traitement liées de manière fonctionnelle dans le sens 5' par rapport à ladite première séquence d'ADN, par laquelle ledit produit d'expression est sécrété.

8. Procédé selon la revendication 6 ou 7 comprenant en outre : l'isolement dudit produit d'expression.
